# EUROPÄISCHE PATENTSCHRIFT

(11) **EP 1 765 428 B1**
(45) Veröffentlichungstag und Bekanntmachung des Hinweises auf die Patenterteilung: **07.01.2015**
(21) Anmeldenummer: 05749138.3
(22) Anmeldetag: 19.05.2005
(51) Int. Cl.: A61L 29/04, A61L 29/18, A61M 25/09

(54) **KATHETER-FÜHRUNGSDRAHT INSBESONDERE FÜR KARDIO-VASKULÄRE EINGRIFFE**
CATHETER GUIDE WIRE, IN PARTICULAR FOR CARDIO-VASCULAR INTERVENTIONS
FIL DE GUIDAGE DE CATHETER NOTAMMENT DESTINE A DES INTERVENTIONS CARDIOVASCULAIRES

(30) Priorität: 11.06.2004 DE 102004028367
(43) Veröffentlichungstag der Anmeldung: 28.03.2007
(73) Patentinhaber: Biotronik VI Patent AG, 6341 Baar (CH)
(72) Erfinder: BILECEN, Deniz, 79650 Schopfheim (DE); HOFMANN, Eugen, CH-8049 Zürich (CH); OSTHEIM-DZEROWYCZ, Wladimir, CH-4125 Riehen (CH)
(74) Vertreter: Lindner-Vogt, Karin L.
(86) Internationale Anmeldenummer: PCT/EP2005/005424
(87) Internationale Veröffentlichungsnummer: WO 2005/120598

(56) Entgegenhaltungen:
- WO-A-97/25093
- WO-A-03/099371
- US-A- 4 182 342
- US-A- 5 209 730
- US-A1- 2003 120 148
- US-A1- 2003 135 114
- US-A1- 2004 087 933
- US-B1- 6 408 214

## Beschreibung

Die Erfindung betrifft einen Katheter-Führungsdraht insbesondere für kardio-vaskuläre Eingriffe gemäß den im Oberbegriff des Anspruches 1 angegebenen Merkmalen.

Zum Hintergrund der Erfindung ist festzuhalten, dass beim Setzen von Kathetern im Zuge von kardio-vaskulären Eingriffen, wie beispielsweise bei der perkutanen transluminalen Angioplastik (PTA) der Katheter mit Hilfe eines vorher in den Körper des Patienten eingeführten Führungsdrahtes positioniert wird. Für die Überwachung des Vorschubes des Führungsdrahtes durch die verschiedenen Blutgefäße bis zu seinem Zielort ist dabei zum einen die Röntgen-Fluoroskopie gängig, zum anderen befindet sich derzeit die Magnetresonanzfluoroskopie in Entwicklung zur Praxisanwendung.

In der Röntgen-Fluoroskopie sind die Führungsdrähte aufgrund ihrer Materialeigenschaften sichtbar. In Spezialfällen, wie beispielsweise für koronare Anwendungen, weisen die Führungsdrähte insbesondere an ihrem distalen Ende radiopaque Marker auf, die sich auf einem Röntgensichtgerät deutlich erkennbar abzeichnen. Diese röntgenologische Sichtbarmachung wird allerdings aufgrund der bekannten nachteiligen Auswirkung von Röntgenstrahlung und der dabei verwendeten Kontrastmittel auf den menschlichen Körper in zunehmendem Maße wohl an Bedeutung verlieren.

Die Magnetresonanztomographie (im Folgenden kurz: MRT) liefert demgegenüber für den Patienten belastungsfrei Bildinformationen mit mehr Gehalt, sofern Katheter-Führungsdrähte eingesetzt werden, die sich in einem Magnetresonanzsichtgerät deutlich abzeichnen.

In dieser Hinsicht ist es beispielsweise aus der WO 98/42268 A1 bekannt, den Führungsdraht mit einer langgestreckten Seele aus einem Material mit hoher spezifischer elektrischer Impedanz, wie beispielsweise einem Glasfasermaterial vorzusehen.

Die WO 03/099371 offenbart einen Führungsdraht mit in seine äußere Beschichtung eingekapseltem radiopaquen Marker. Der Führungsdraht weist am Ende eine Verjüngung auf Zusätzlich ist eine gleitfähige hydrophile Schicht aufgebracht.

Ferner ist es aus den beiden als Stamm- und Fortsetzungsanmeldung miteinander verknüpften Druckschriften US 2003/0120148 A1 und US 2003/0135114 A1 bekannt, als Seele einen langgestreckten PEEK-Kern zu verwenden. Dieser erstreckt sich vom proximalen Ende des Führungsdrahtes ausgehend bis vor dessen distales Ende und geht dort in einen distalen Endabschnitt über, der aus einem nicht magnetischen Metall- oder Legierungsmaterial besteht. Dieses soll flexibler sein als der PEEK-Kern des proximalen Kernabschnittes des Katheter-Führungsdrahtes.

Der in den beiden vorgenannten Druckschriften gezeigte Führungsdraht ist ferner an seinem distalen Ende mit einer metallischen, jedoch zumindest teilweise aus nichtmagnetischem Material gefertigten Wendel versehen, die einen hohen Kontrast in einem MRT-Gerät liefert.

Insgesamt ist der vorbekannte Führungsdraht mit seiner mehrteiligen Seele konstruktiv äußerst aufwändig gestaltet. Insoweit liegt der Erfindung die Aufgabe zugrunde, einen magnetresonanz-kompatiblen Führungsdraht zu schaffen, der sich durch einen einfachen Aufbau bei hoher Flexibilität des distalen Endes und guter Erkennbarkeit in MRT-Geräten auszeichnet.

Diese Aufgabe wird durch die im Kennzeichnungsteil des Anspruches 1 angegebenen Merkmale gelöst. Demnach ist der Kern des Führungsdrahtes einstückig vom proximalen bis zum distalen Ende durchgehend aus PEEK-Material gebildet und im Endbereich zu seinem distalen Ende im Durchmesser verjüngt ausgestaltet, läuft also im Wesentlichen "zugespitzt" aus. Ferner ist der Führungsdraht mit einer Beschichtung versehen, in die ein MRT-Kontrastmittel inkorporiert ist. Gängige MRT-Kontrastmittel sind Elemente der Lanthaniden-Gruppe, wie Gadolinium oder Dysprosium.

Auch die Einlagerung von Titan-Partikeln als MRT-Kontrastmittel in die Beschichtung ist denkbar. Bevorzugter Maßen wird in die Beschichtung Eisenpulver als Kontrastmittel integriert.

Da die Beschichtung ferner die Aufgabe hat, für den Führungsdraht gute Gleiteigenschaften beim Einführen in ein Körpergefäß und für das Überschieben eines Katheters zu gewährleisten, erfüllt diese Beschichtung in vorteilhafter Weise also eine typische Doppelfunktion.

Es hat sich herausgestellt, dass ein Führungsdraht mit einem durchgehenden PEEK-Kern und der angegebenen Beschichtung sich in MRT-Geräten deutlich abzeichnet. Dies trifft auch mit der für eine Erhöhung der Flexibilität des distalen Endes vorgesehenen Verjüngung des Kerns an diesem Ende zu. Insgesamt weist der erfindungsgemäße Katheter-Führungsdraht eine gute MRT-Kompatibilität auf, da er allenfalls geringe Artefakte in MRT-Sichtgeräten zeigt. Es findet insbesondere keine Erwärmung des Führungsdrahtes statt, wie dies bei Einsatz von röntgen-kompatiblen Führungsdrähten in MRT-Sichtgeräten der Fall ist.

Aufgrund der Verjüngung des Kerns zum distalen Ende hin wird die geforderte Flexibilität des distalen Endes zum Vorschieben des Führungsdrahtes auch um engere Biegungen, durch entsprechende filigrane Abzweigungen und Passagen im kardio-vaskulären System gewährleistet.

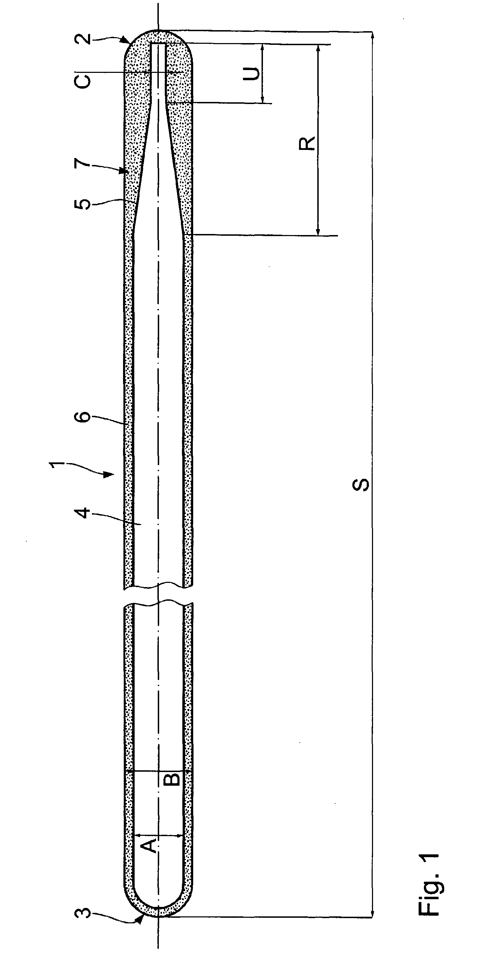

Bevorzugte Ausführungsformen der Erfindung sowie weitere Merkmale, Einzelheiten und Vorteile sind den Unteransprüchen und der nachfolgenden Beschreibung entnehmbar, in der ein Ausführungsbeispiel eines Katheter-Führungsdrahtes anhand der beigefügten Zeichnung näher erläutert wird. Diese
- Fig. 1: zeigt einen schematischen Längsaxialschnitt eines Katheter-Führungsdrahtes.

Der in der Zeichnung als Ganzes mit 1 bezeichnete Katheter-Führungsdraht - die Bezeichnung "Draht" ist trotz der Tatsache, dass der Gegenstand nicht aus Metall besteht, in der Medizintechnik üblich - weist ein in den Körper eines Patienten einzuführendes distales Ende 2 und ein proximales Ende 3 auf, das zur Handhabung des Führungsdrahtes 1 außerhalb des Körpers verbleibt. Tragendes Teil des Führungsdrahtes 1 ist ein Kern 4, der als langgestrecktes Polyetheretherketon- (PEEK-) Monofilament ausgebildet ist. Bis auf einen kurzen Endbereich R von etwa 100 mm vor dem distalen Ende 2 weist der Kern 4 einen konstanten Durchmesser A von etwa 0,7 mm auf. Im Endbereich R ist eine konische Verjüngung 5 zum distalen Ende 2 hin vorgesehen, in deren Verlauf sich der Durchmesser des Kerns 4 auf einen Wert C von beispielsweise 0,25 mm reduziert. In einem kurzen Spitzenabschnitt U von beispielsweise 20 mm Länge weist der Kern 4 diesen konstanten Außendurchmesser C auf. Die Gesamtlänge S des Katheter-Führungsdrahtes ist an den jeweiligen Patienten anzupassen und kann beispielsweise 1500 mm betragen.

Der Führungsdraht 1 ist über die gesamte Oberfläche seines Kerns 4 mit einer ein- oder mehrkomponentigen Beschichtung 6 versehen, die im Bereich des konstanten Durchmessers A eine Dicke von etwa 0,07 mm aufweisen kann. Der Gesamt-Außendurchmesser B des Führungsdrahtes beträgt somit 0,85 mm und bleibt über die gesamte Länge, also auch im distalen Endbereich R konstant. Dementsprechend nimmt, wie in der Zeichnung gut erkennbar ist, die Beschichtungsdicke im distalen Endbereich R entsprechend der Durchmesserverringerung der Seele 4 zu.

Die für die gegebenenfalls mehrlagige Beschichtung 6 einsetzbaren Materialien müssen in erster Linie reibungsvermindernd dahingehend sein, dass das Einführen des Führungsdrahtes in Körpergefäße und das Überschieben des Katheters über den Führungsdraht möglichst leichtgängig erfolgen können. Einschlägige Beschichtungsmaterialien sind Polyurethane und Polyamide, die als innere Lage der Beschichtung eingesetzt werden können. Für die äußere Lage kommen hydrophile, Silikon- oder Polysiloxan-Beschichtungen zur Reibungsverminderung in Frage. Bei der Auslegung des Materials der Beschichtung 6 ist darauf zu achten, dass die Seele 4 demgegenüber eine höhere Biegesteifigkeit aufweist, damit der distale Endbereich R eine Erhöhung seiner Flexibilität aufgrund der Durchmesserverringerung der Seele erfährt.

Wie in der Zeichnung höchst schematisch und nur in einem Teilbereich durch eine Punktierung angedeutet ist, ist die Beschichtung 6 über ihr gesamtes Volumen mit Eisenpulver 7 versetzt, das eine Korngröße von unter 10 µm aufweist. Sein Mengenanteil bezogen auf die Beschichtung beträgt etwa 1 bis 2 Gew.%. Das Eisenpulver 7 in der Beschichtung 6 führt zu einer verbesserten Erkennbarkeit des Führungsdrahtes sowohl in Röntgenstrahlungs- als auch MRT-Geräten, da es einerseits sich als Röntgenkontrastmittel gut abzeichnet und andererseits auf einem MRT-Gerät einen sogenannten "Auslöschungs-Effekt" erzeugt. Obwohl dies an sich ein Störeffekt ist, kann dadurch der Führungsdraht durch MRT-Detektion gut erkannt werden. Durch die Dickenzunahme der Beschichtung 6 am distalen Ende 2 und die damit verbundene höhere Menge an Eisenpulver 7 in diesem Drahtbereich ist dessen Endbereich R auch besonders gut erkennbar.

Zum Herstellungsvorgang für den Katheter-Führungsdraht ist festzuhalten, dass der PEEK-Kern extrudiert, anschließend durch Recken geradegerichtet und schließlich im distalen Endbereich R abgeschliffen wird.

## Patentansprüche

1. Katheter-Führungsdraht, insbesondere für kardio-vaskuläre Eingriffe, umfassend
- ein distales Ende (2) und ein proximales Ende (3),
- einen langgestreckten PEEK-Kern (4), und
- eine Beschichtung (6) auf dem Kern (4),
**dadurch gekennzeichnet, dass**
- der PEEK-Kern (4) vom proximalen (3) bis zum distalen Ende (2) des Führungsdrahtes (1) einstückig durchgehend ausgeführt ist,
- der Kern (4) von einem Proximaldurchmesser (A) sich im Endbereich (R) zu ihrem distalen Ende (3) hin auf einen Distaldurchmesser (C) verjüngt, und
- die Beschichtung (6) mit einem MRT-Kontrastmittel (7) versetzt ist.

2. Katheter-Führungsdraht nach Anspruch 1, **dadurch gekennzeichnet, dass** die Beschichtung (6) mit einem sowohl Röntgenstrahlungs- als auch Magnetresonanzdetektierbaren Kontrastmittel (7) versetzt ist.

3. Katheter-Führungsdraht nach Anspruch 2, **gekennzeichnet durch** Eisenpulver (7) vorzugsweise mit einer Korngröße von unter 10 µm als Kontrastmittel.

4. Katheter-Führungsdraht nach Anspruch 3, **dadurch gekennzeichnet, dass** das Eisenpulver (7) in einer Menge von 1 bis 2 Gew.% bezogen auf die Beschichtung (6) eingesetzt ist.

5. Katheter-Führungsdraht nach einem der vorangehenden Ansprüche, **dadurch gekennzeichnet, dass** der Kern (4) eine höhere Biegesteifigkeit aufweist als die Beschichtung (6).

6. Katheter-Führungsdraht nach einem der vorangehenden Ansprüche, **dadurch gekennzeichnet, dass** der Kern (4) aus einem extrudierten PEEK-Monofilament besteht.

7. Katheter-Führungsdraht nach einem der vorgenannten Ansprüche, **dadurch gekennzeichnet, dass** die Beschichtung (6) im Endbereich (R) derart in ihrer Dicke zunimmt, dass der Außendurchmesser (B) des Führungsdrahtes (1) über seine Länge (S) im Wesentlichen konstant ist.

8. Katheter-Führungsdraht nach einem der vorgenannten Ansprüche, **dadurch gekennzeichnet, dass** Proximal- (A) zu Distaldurchmesser (C) der Seele (4) ein Verhältnis von etwa 2:1 bis 5:1, vorzugsweise etwa 3:1 aufweisen.

9. Katheter-Führungsdraht nach einem der vorgenannten Ansprüche, **dadurch gekennzeichnet, dass** die Beschichtung (6) eine reibungsvermindernde Polymerlage aufweist.

10. Katheter-Führungsdraht nach Anspruch 6, **dadurch gekennzeichnet, dass** die Beschichtung (6) mit einer hydrophilen Außenlage versehen ist.

## Claims

1. Catheter guide wire, in particular for cardiovascular interventions, comprising
- a distal end (2) and a proximal end (3),
- an elongated PEEK core (4), and
- a coating (6) on the core (4),
**characterized in that**
- the PEEK core (4) is designed as one continuous piece from the proximal end (3) to the distal end (2) of the guide wire (1),
- the core (4) tapers from a proximal diameter (A) in the end region (R) toward the distal end (3) thereof to a distal diameter (C), and
- the coating (6) contains an MRT contrast medium (7).

2. The catheter guide wire according to claim 1, **characterized in that** the coating (6) contains a contrast medium (7) that can be detected by x-rays and by magnetic resonance.

3. The catheter guide wire according to claim 2, **characterized by** iron powder (7) preferably having a particle size of less than 10 µm, as the contrast medium.

4. The catheter guide wire according to claim 3, **characterized in that** the iron powder (7) is used in a quantity of 1 to 2% by weight relative to the coating (6).

5. The catheter guide wire according to any one of the preceding claims, **characterized in that** the core (4) has greater bending resistance than the coating (6).

6. The catheter guide wire according to any one of the preceding claims, **characterized in that** the core (4) is made of an extruded PEEK monofilament.

7. The catheter guide wire according to any one of the preceding claims, **characterized in that** the coating (6) increases in thickness in the end region (R) such that the outer diameter (B) of the guide wire (1) is substantially constant along the length (S) thereof.

8. The catheter guide wire according to any one of the preceding claims, **characterized in that** the proximal diameter (A) to the distal diameter (C) of the core (4) has a ratio of approximately 2:1 to 5:1, preferably approximately 3:1.

9. The catheter guide wire according to any one of the preceding claims, **characterized in that** the coating (6) has a friction-reducing polymer layer.

10. The catheter guide wire according to claim 6, **characterized in that** the coating (6) is provided with a hydrophilic outer layer.

## Revendications

1. Fil de guidage pour cathéter, en particulier pour des interventions cardiovasculaires, comprenant
- une extrémité distale (2) et une extrémité proximale (3),
- un noyau en PEEK (4) allongé, et
- un revêtement (6) sur le noyau (4),
**caractérisé en ce que**
- le noyau en PEEK (4) est conçu d'une seule pièce continue, de l'extrémité distale (3) jusqu'à l'extrémité proximale (2) du fil de guidage (1),
- le noyau (4) s'affine à partir d'un diamètre proximal (A) dans la région terminale (R) vers son extrémité distale (3) jusqu'à un diamètre distal (C), et
- le revêtement (6) comprend un produit de contraste pour IRM (7).

2. Fil de guidage pour cathéter selon la revendication 1, **caractérisé en ce que** le revêtement (6) comprend un produit de contraste (7) détectable aussi bien par un rayonnement X que par résonance magnétique.

3. Fil de guidage pour cathéter selon la revendication 2, **caractérisé par** une poudre de fer (7), de préférence avec une taille de grain inférieure à 10 µm en tant que produit de contraste.

4. Fil de guidage pour cathéter selon la revendication 3, **caractérisé en ce que** la poudre de fer (7) est utilisée dans une quantité de 1 à 2% en poids par rapport au revêtement (6).

5. Fil de guidage pour cathéter selon la revendication 3, **caractérisé en ce que** le noyau (4) présente une plus grande rigidité à la flexion que le revêtement (6).

6. Fil de guidage pour cathéter selon l'une des revendications précédentes, **caractérisé en ce que** le noyau (4) est constitué d'un monofilament de PEEK extrudé.

7. Fil de guidage pour cathéter selon l'une des revendications précédentes, **caractérisé en ce que** dans la région terminale (R), l'épaisseur du revêtement (6) augmente de manière à ce que le diamètre extérieur (B) du fil de guidage (1) soit quasiment constant sur sa longueur (S).

8. Fil de guidage pour cathéter selon l'une des revendications précédentes, **caractérisé en ce que** le rapport du diamètre proximal (A) au diamètre distal (C) de l'âme (4) est d'environ 2:1 à 5:1, de préférence d'environ 3:1.

9. Fil de guidage pour cathéter selon l'une des revendications précédentes, **caractérisé en ce que** le revêtement (6) comporte une couche polymère réduisant la friction.

10. Fil de guidage pour cathéter selon la revendication 6, **caractérisé en ce que** le revêtement (6) est pourvu d'une couche extérieure hydrophile.
